# FASCICULE DE BREVET EUROPEEN

(11) **EP 1 189 617 B1**
(45) Date de publication et mention de la délivrance du brevet: **27.08.2003**
(21) Numéro de dépôt: 00949569.8
(22) Date de dépôt: 29.06.2000
(51) Int. Cl.: A61K 31/505, A61K 31/165

(54) **ASSOCIATION PHARMACEUTIQUE A ACTIVITE ANALGESIQUE CONTENANT DU PARACETAMOL ET DE LA BUSPIRONE**
PARACETAMOL UND BUSPIRON ENTHALTENDE PHARMAZEUTISCHE ZUSAMMENSETZUNG MIT ANALGETISCHER WIRKUNG
PHARMACEUTICAL COMBINATION WITH ANALGESIC ACTION CONTAINING PARACETAMOL AND BUSPIRONE

(30) Priorité: 30.06.1999 FR 9908363
(43) Date de publication de la demande: 27.03.2002
(73) Titulaire: LABORATOIRES UPSA, 47000 Agen (FR)
(72) Inventeur: CAMBORDE, Françoise, F-91400 Orsay (FR); CLOAREC, Alix, F-78510 Triel sur Seine (FR)
(74) Mandataire: Hubert, Philippe
(86) Numéro de dépôt international: FR0001817
(87) Numéro de publication internationale: WO01001989

(56) Documents cités:
- US-A- 4 435 405
- US-A- 4 576 953
- US-A- 4 607 039

## Description

La présente invention a pour objet une nouvelle association pharmaceutique trouvant notamment application dans le traitement de la douleur.

Plus précisément, l'invention concerne une association pharmaceutique comprenant, à titre de principes actifs associés, d'une part du p-acétamidophénol connu sous le nom de paracétamol ou d'acetaminophen et d'autre part de la 8-[4-[4-(2-pyrimidinyl-1-piperazin-yl]butyl]-8-azaspiro[4,5]decane-7,9-dione, connue sous le nom de buspirone, lesdits principes actifs étant conditionnés soit ensemble au sein d'une préparation pharmaceutique unique, soit séparément au sein de deux préparations pharmaceutiques destinées à être administrées simultanément.

Le paracétamol est couramment utilisé aujourd'hui comme analgésique et antipyrétique. Les propriétés analgésiques sont mises en évidence dans différents modèles expérimentaux de douleurs.

### Paracétamol

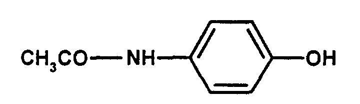

La buspirone, sous forme de chlorhydrate, est utilisée en thérapeutique comme anxiolytique.

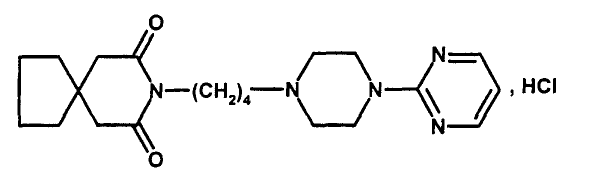

### Chlorhydrate de buspirone

On peut trouver sa méthode de préparation dans la littérature (Y.H. Wu *et al.* J. Med. Chem 1972 ; 15 : 477 / US patent : 3, 717, 634).

Bien que le mécanisme d'action de la buspirone ne soit pas complètement élucidé, il semble que son activité relève essentiellement de ses effets sur les récepteurs sérotoninergiques. Elle agit principalement en tant qu'agoniste des récepteurs 5-HT1A présynaptiques et agoniste partiel des récepteurs 5-HT1A post-synaptiques. Elle possède également une activité antagoniste des récepteurs dopaminergiques D2 essentiellement présynaptiques.

Il a été découvert, et ceci constitue le fondement de la présente invention, que l'association du paracétamol et de la buspirone présente un effet analgésique significatif, à des doses où chacun des produits constitutifs de cette association est inactif ou très peu actif.

L'effet bénéfique de l'association, conforme à la présente invention, a été démontré pour un modèle de douleur aiguë.

Les résultats obtenus ont montré que cette association présente une activité analgésique supérieure à celle obtenue avec le paracétamol seul.

L'effet de potentialisation ainsi démontré rend possible l'utilisation de faibles doses de chacun des produits constitutifs de l'association en limitant ainsi les possibles effets secondaires, ainsi que le traitement de douleurs intenses où le paracétamol seul montre une activité faible, voire nulle.

De plus, cette association permet le traitement de douleurs d'origines très variées chez un plus grand nombre de patients.

Dans l'association pharmaceutique conforme à la présente invention, le rapport pondéral du paracétamol à la buspirone sera choisi pour conduire à la meilleure synergie entre les deux composés associés ; il sera compris d'une façon générale entre 3:1 et 30:1 et sera de préférence de l'ordre de 10:1.

La dose journalière utilisable des différents composés constituant l'association pharmaceutique conforme à l'invention dépendra bien entendu, de l'état du patient à traiter.

Une dose journalière appropriée de paracétamol sera généralement comprise entre 300 mg et 1000 mg.

Avantageusement, l'association pharmaceutique conforme à la présente invention se présentera sous une forme appropriée pour une administration :
- par voie orale, comme par exemple, sous forme de comprimés simples ou dragéifiés, de gélules ou de granulés ;
- par voie rectale, comme par exemple, sous forme de suppositoires ;
- par voie parentérale, comme par exemple, sous forme de préparations injectables ;
- par voie oculaire, comme par exemple, sous forme de collyres ou de solutions ophtalmiques ;
- par voie transdermique ;
- par voie nasale, comme par exemple, sous forme d'aérosols et sprays ;
- par voie auriculaire, comme par exemple, sous forme de gouttes.

Les principes actifs préférés peuvent être utilisés sous forme libre ou sous la forme d'un sel pharmaceutiquement acceptable.

Selon un mode de réalisation actuellement préféré, l'association pharmaceutique conforme à l'invention se présentera sous la forme d'une préparation pharmaceutique unique incorporant les deux principes actifs.

Une telle formulation peut être préparée, selon des méthodes connues en soi, en incorporant les principes actifs, constituant l'association précitée, à des excipients habituellement utilisés tels que le talc, la gomme arabique, le lactose, l'amidon, le stéarate de magnésium, la polyvidone, les dérivés de la cellulose, le beurre de cacao, les glycérides semi-synthétiques, les véhicules aqueux ou non, les corps gras d'origine animale ou végétale, les glycols, les agents mouillants, dispersants ou émulsifiants, les gels de silicone, certains polymères ou copolymères, les conservateurs, arômes et colorants.

Selon un second mode de réalisation de l'invention, les principes actifs constituant l'association précitée seront conditionnés séparément, au sein de deux formulations, de préférence appropriées pour une même voie d'administration, destinées à être administrées simultanément.

Avantageusement, les associations pharmaceutiques selon l'invention se présenteront sous forme de doses unitaires.

Comme exemple de doses unitaires convenant dans le cadre de la présente invention, on peut citer celles contenant de 50 à 200 mg de paracétamol et/ou de 5 à 20 mg de buspirone.

Les associations pharmaceutiques conformes à la présente invention conviennent au traitement de la douleur.

On peut citer par exemple leur utilisation dans le traitement des douleurs articulaires et en particulier de l'arthrite, de l'arthrite rhumatoïde, de la spondylarthrite, de l'arthrite de la goutte, de l'ostéoarthrite, et de l'arthrite juvénile.

Ces associations peuvent également être utilisées dans le cadre du traitement des dysménorrhées, des tendinites et des bursites. Elles peuvent aussi être utilisées dans le traitement des symptômes douloureux des algies musculaires, des douleurs dentaires, des migraines, des douleurs d'origine cancéreuse, ainsi qu'à titre de traitement complémentaire dans les états infectieux et fébriles.

Ces compositions peuvent enfin être proposées dans le traitement des douleurs neuropathiques et en particulier des algies nerveuses, des zonas, des douleurs de désafférentation, des neuropathies diabétiques.

### Mise en évidence des propriétés analgésiques de l'association pharmaceutique conforme à l'invention

Pour mettre en évidence les propriétés analgésiques spécifiques de l'association pharmaceutique conforme à la présente invention, on a réalisé un essai pharmacologique dont le protocole expérimental et les résultats seront donnés ci-après.

### Essai utilisé : test de la plaque chauffante

Le test est réalisé en suivant le protocole expérimental décrit par Eddy, N.B., C.F. Toucheberry, J.E. Lieberman. Synthetic analgesics. 1 - Methadone isomers and derivatives, J. Pharmacol. Exp. Ther. 1950 ; (98) : 121-137.

La souris déposée sur une plaque chauffée à 52°C ± 0,05 manifeste sa douleur par le léchage des pattes antérieures ou plus rarement par un saut.

Le temps de réaction est alors noté, le temps maximum étant de 30 secondes.

Les composés ou association étudiés sont administrés par voie intrapéritonéale, une demi-heure avant le test.

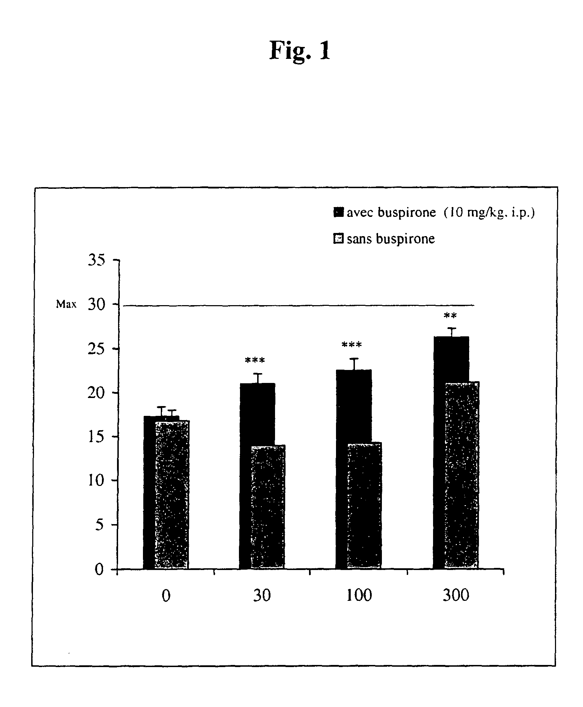

Les résultats obtenus sont représentés à la figure 1 ci-jointe qui montre clairement l'effet de potentialisation exercé par la buspirone sur le paracétamol.

Sur cette figure, on a représenté en abscisse, la quantité de paracétamol exprimée en mg par kilo et en ordonnée le temps d'apparition de la réaction douloureuse exprimée en secondes.

Les résultats obtenus sans buspirone sont représentés en gris clair, tandis que ceux obtenus avec buspirone sont représentés en gris foncé.

On donnera maintenant plusieurs exemples de formulations pharmaceutiques selon l'invention :

### EXEMPLES D'ASSOCIATIONS PARACETAMOL/BUSPIRONE

| **Exemple 1 :** Gélule (taille n° 1) | | |
|---|---|---|
| Paracétamol | 100 mg | |
| Buspirone | 10 mg | |
| Cellulose microcristalline | 100 mg | |
| Hydroxypropylméthylcellulose | 10 mg | |
| Stéarate de magnésium | 5 mg | pour une gélule |

| **Exemple 2 :** Comprimé | | |
|---|---|---|
| Paracétamol | 100 mg | |
| Buspirone | 10 mg | |
| Cellulose microcristalline | 100 mg | |
| Lactose | 100 mg | |
| Hydroxypropylméthylcellulose | 10 mg | |
| Stéarate de magnésium | 5 mg | |
| Hydroxypropylcellulose | 50 mg | pour un comprimé |

| **Exemple 3 :** Suppositoire | | |
|---|---|---|
| Paracétamol | 200 mg | |
| Buspirone | 20 mg | |
| Glycéride semi-synthétique | 1900 mg | pour un suppositoire |
| (suppocire) | | |

| **Exemple 4 :** Solution ophtalmique | |
|---|---|
| Paracétamol | 500 mg |
| Buspirone | 50 mg |
| Parahydroxybenzoate de méthyle sodé | 140 mg |
| Huile de ricin (Cremophor EL) | 5 mg |
| Polysorbate 80 | 10 mg |
| Eau purifiée q.s.p. | 100 ml |

| **Exemple 5 :** Préparation injectable | |
|---|---|
| Paracétamol | 500 mg |
| Buspirone | 50 mg |
| Cystéine | 50 mg |
| PEG 400 | 30 mg |
| Alcool éthylique | 10 mg |
| Eau ppi q.s.p. | 100 ml |

Pour l'utilisation en prise séparée et simultanée des deux principes actifs, les compositions sont conformes à celles déjà utilisées pour chacun des produits en choisissant les dosages adaptés.

## Revendications

1. Association pharmaceutique, **caractérisée en ce qu'**elle comprend à titre de principes actifs associés, du paracétamol et de la buspirone ou l'un de ses sels pharmaceutiquement acceptables.

2. Association pharmaceutique selon la revendication 1, **caractérisée en ce qu'**elle se présente sous une forme appropriée pour une administration par voie orale, par voie parentérale, par voie rectale, par voie oculaire, par voie transdermique, par voie nasale ou par voie auriculaire.

3. Association pharmaceutique selon l'une des revendications 1 ou 2, **caractérisée en ce que** le rapport pondéral du paracétamol à la buspirone est compris entre 3:1 et 30:1 et sera de préférence de l'ordre de 10:1.

4. Association pharmaceutique selon la revendication 1, **caractérisée en ce que** les principes actifs associés précités sont conditionnés ensemble au sein d'une préparation unique.

5. Association pharmaceutique selon la revendication 4, **caractérisée en ce qu'**elle se présente sous forme d'une dose unitaire contenant de 50 à 200 mg de paracétamol et de 5 à 20 mg de buspirone.

6. Association pharmaceutique selon la revendication 1, **caractérisée en ce que** les principes actifs associés précités sont conditionnés séparément au sein de deux préparations distinctes, lesdites préparations étant destinées à être administrées simultanément.

7. Association pharmaceutique selon la revendication 6, **caractérisée en ce qu'**elle se présente sous forme de deux doses unitaires contenant l'une de 50 à 200 mg de paracétamol, l'autre de 5 à 20 mg de buspirone.

## Patentansprüche

1. Arzneimittelkombination, **dadurch gekennzeichnet, dass** sie als kombinierte Wirkstoffe Paracetamol und Buspiron oder eines seiner pharmazeutisch verträglichen Salze umfasst.

2. Arzneimittelkombination gemäß Anspruch 1, **dadurch gekennzeichnet, dass** sie in einer geeigneten Form für eine Verabreichung auf oralem Wege, auf parenteralem Wege, auf rectalem Wege, auf okularem Wege, auf transdermalem Wege, auf nasalem Wege oder auf aurikularem Wege vorliegt.

3. Arzneimittelkombination gemäß einem der Ansprüche 1 oder 2, **dadurch gekennzeichnet, dass** das Gewichtsverhältnis von Paracetamol zu Buspiron zwischen 3:1 und 30:1 und vorzugsweise in der Größenordnung von 10:1 liegt.

4. Arzneimittelkombination gemäß Anspruch 1, **dadurch gekennzeichnet, dass** die kombinierten Wirkstoffe zusammen innerhalb einer einzigen Formulierung vorliegen.

5. Arzneimittelkombination gemäß Anspruch 4, **dadurch gekennzeichnet, dass** sie in Form einer Einheitsdosis vorliegt, die 50 bis 200 mg Paracetamol und 5 bis 20 mg Buspiron enthält.

6. Arzneimittelkombination gemäß Anspruch 1, **dadurch gekennzeichnet, dass** die kombinierten Wirkstoffe getrennt voneinander innerhalb von zwei unterschiedlichen Formulierungen vorliegen, wobei die Formulierungen dazu vorgesehen sind, gleichzeitig verabreicht zu werden.

7. Arzneimittelkombination gemäß Anspruch 6, **dadurch gekennzeichnet, dass** sie in Form von zwei Einheitsdosen vorliegt, von denen die eine 50 bis 200 mg Paracetamol, die andere 5 bis 20 mg Bispiron enthält.

## Claims

1. A pharmaceutical combination, **characterised in that** it comprises, as combined active principles, paracetamol and buspirone or one of its pharmaceutically acceptable salts.

2. The pharmaceutical combination according to claim 1, **characterised in that** it is presented in a form suitable for an administration *via* the oral route, *via* the parenteral route, *via* the rectal route, *via* the ocular route, *via* the transdermal route, *via* the nasal route, or *via* the auricular route.

3. The pharmaceutical combination according to one of claims 1 or 2, **characterised in that** the paracetamol to buspirone weight ratio is between 3:1, and 30:1, and will more preferably be of the order of 10:1.

4. The pharmaceutical combination according to claim 1, **characterised in that** the combined active principles mentioned above are packaged together in a single preparation.

5. The pharmaceutical combination according to claim 4, **characterised in that** it is presented in the form of a unit dose containing 50 to 200 mg of paracetamol and 5 to 20 mg of buspirone.

6. The pharmaceutical combination according to claim 1, **characterised in that** the combined active principles mentioned above are packaged separately in two separate preparations, said preparations intended to be administered simultaneously.

7. The pharmaceutical combination according to claim 6, **characterised in that** it is presented in the form of two unit doses, one containing 50 to 200 mg of paracetamol, and the other 5 to 20 mg of buspirone.
